# EUROPEAN PATENT SPECIFICATION

(11) **EP 0 581 613 B1**
(45) Date of publication and mention of the grant of the patent: **11.09.1996**
(21) Application number: 93306069.1
(22) Date of filing: 30.07.1993
(51) Int. Cl.: C07C 239/20, C07C 271/12, C07C 263/00

(54) **Process for preparing N,O-dialkylhydroxycarbamic acid esters**
Verfahren zur Herstellung von N,O-Dialkylhydroxycarbaminsäureestern
Procédé de préparation d'esters d'acides carbamiques N,O-dialkylhydroxylés

(30) Priority: 31.07.1992 JP 205804/92; 14.10.1992 JP 276381/92; 23.10.1992 JP 286283/92; 26.03.1993 JP 68664/93; 30.06.1993 JP 162981/93
(43) Date of publication of application: 02.02.1994
(60) Divisional application: 95115614.0
(73) Proprietor: MITSUI PETROCHEMICAL INDUSTRIES, LTD., Tokyo (JP)
(72) Inventor: Inoki, Satoshi, c/o Mitsui Petrochem. Ind., Ltd., Kuga-gun, Yamaguchi-ken (JP); Takesue, Mitsuyuki, c/o Mitsui Petrochem. Ind. Ltd, Kuga-gun, Yamaguchi-ken (JP); Hashimoto, Isao, c/o Mitsui Petrochemical Ind. Ltd, Kuga-gun, Yamaguchi-ken (JP); Kihara, Noriaki, c/o Mitsui Petrochem. Ind., Ltd., Kuga-gun, Yamaguchi-ken (JP); Sugi, Kiyoaki, c/o Mitsui Petrochem. Ind., Ltd., Kuga-gun, Yamaguchi-ken (JP)
(74) Representative: Cresswell, Thomas Anthony

(56) References cited:
- DE-A- 3 245 503
- GB-A- 1 042 191
- US-A- 3 230 260
- TETRAHEDRON LETTERS vol. 30, no. 1, 1989, pages 31 - 34 R. SULSKY ET AL
- CHEMICAL ABSTRACTS, vol. 81, 1974, Columbus, Ohio, US; abstract no. 151392c,

## Description

This invention relates to a process for preparing N,O-dialkylhydroxycarbamic acid esters, used as intermediates in the synthesis of N,O-dialkylhydroxylamines which themselves are intermediates in the synthesis of pharmaceuticals and agricultural chemicals.

A generally known process for preparing N,O-dialkylhydroxycarbamic acid esters from hydroxylamine or its salts comprises reacting chloroformic acid ester with a hydroxylamine salt in the presence of sodium hydroxide and subsequently reacting the reaction solution with a dialkyl sulfate in the presence of sodium hydroxide.

For example, Org. Prep. Proced. [Vol. 19, P. 75 (1987)] discloses a process for preparing ethyl N,O-dimethylhydroxycarbamate. According to this process, hydroxylamine hydrochloride is reacted with ethyl chloroformate using a sodium hydroxide solution to obtain ethyl hydroxycarbamate, and then this product is dimethylated with dimethyl sulfate and a sodium hydroxide solution to obtain ethyl N,O-dimethylhydroxycarbamate in a 70 - 73% yield.

With respect to butyl N,O-dimethylcarbamate DE-A-3,245,503 discloses a process which comprises reacting hydroxylamine sulfate as a starting material with butyl chloroformate in the presence of sodium hydroxide, extracting the reaction product with dichloromethane, drying the extract, distilling the solvent off from the dried extract to obtain butyl hydroxycarbamate and dimethylating this product using dimethyl sulfate and a sodium hydroxide solution to obtain ethyl N,O-dimethylhydroxycarbamate in a total yield of 65%.

However, the processes which had been reported so far are disadvantageous in that they give the desired N,O-dialkylhydroxycarbamic acid ester in low yields when applied industrially. In addition, the above processes, according to studies by the present inventors, have been found to be disadvantageous in the case of carbamoylating hydroxylamine or its salts with methyl chloroformate and then dialkylating the resulting product to prepare N,O-dialkylhydroxycarbamate. The methyl chloroformate used as a carbamoylating agent is liable to be hydrolyzed because this reaction is carried out using an aqueous solution as a solvent. Furthermore the yield of the desired product becomes even lower because the hydroxyl group of the methyl hydroxycarbamate formed as an intermediate reacts with methyl chloroformate, as shown in Comparative Example 2 (see late).

N,O-dialkylhydroxylamine can be prepared from the N,O-dialkylhydroxycarbamic acid ester by hydrolyzing the ester in the presence of hydrochloric acid as a catalyst in an aqeuous solution, as disclosed, for example, in DE-A-3,245,503; Org. Prep. Proced.[Vol. 19, P. 75 (1987)] discloses the hydrolysis of ethyl N,O-dimethylhydroxycarbamate with hydrochloric acid and azeotropically dehydrating the hydrolysate with isopropanol to obtain N,O-dimethylhydroxylamine hydrochloride. In order to recover the obtained N,O-dimethylhydroxylamine hydrochloride in an anhydrous state, a complicated post-treatment is disclosed in Org. Prep. Proced. [Vol. 19, P. 75 (1987)].is required. Therefore, these cannot be industrial processes for preparation. Tett. Letts., 30, 31-34 (1989) discloses a process wherein an unsubstituted carbamate is prepared from di-t.-butyl dicarbonate and O-benzyl hydroxylamine in aqueous base. Treatment of the carbamate with sodium hydride followed by the addition of a primary halide or secondary mesylate or iodide gives the t-butyl carbamate.

The present invention seeks to provide a process for preparing an N,O-dialkylhydroxycarbamic acid ester in yields higher than conventional processes.

The present invention provides a process of preparing an N,O-dialkylhydroxycarbamic acid ester of formula (III) 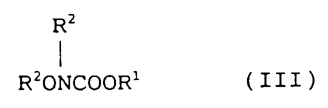 wherein R¹ is a hydrocarbon group, and R² is lower alkyl, said process comprising reacting hydroxylamine of formula NH₂OH or a salt thereof with a dihydrocarbyl carbonate of formula (I)

R¹OCOOR¹ (I)

wherein R¹ is as defined above,
in the presence of a base, and alkylating the resultant hydroxy-carbamic acid ester of formula (II)

HONHCOOR¹ (II)

wherein R¹ is as defined above,
with an alkylating agent in the presence of a base.

For example, the dihydrocarbyl carbonate is dimethyl carbonate, the hydroxycarbamic acid ester is methyl hydroxycarbamate and the N,O-dialkylhydroxycarbamic acid ester is methyl N,O-dimethylhydroxycarbamate.

The preferred reaction temperatures in the carbamoylation and dialkylation are 5 ± 5°C and the pHs of the reaction solutions in the reactions are preferably 12 - 13.

The N,O-dialkylhydroxycarbamic acid ester may be recovered from the solution obtained by azeotropic distillation with water.

Examples of the hydrocarbon group represented by R¹ are C₁-C₅ alkyl groups such as a methyl group, an ethyl group, a n-propyl group, an isopropyl group, a n-butyl group, an isobutyl group, a sec-butyl group, a tert-butyl group or a n-pentyl group, preferably C₁-C₃ alkyl groups such as a methyl group, an ethyl group, a n-propyl group or an isopropyl group; C₃-C₇ cycloalkyl groups such as a cyclopropyl group, a cyclobutyl group, a cyclopentyl group, a cyclohexyl group or a cycloheptyl group ; aryl groups such as a phenyl group, a tolyl group or a xylyl group ; and aralkyl groups such as a benzyl group or a phenethyl group.

The lower alkyl group represented by R² is a C₁-C₄ alkyl group, for example a methyl group, an ethyl group, a n-propyl group, an isopropyl group, a n-butyl group, an isobutyl group, a sec-butyl group or a tert-butyl group.

Examples of the hydroxylamine or the salt thereof used as a starting material are hydroxylamine, hydroxylamine sulfate and hydroxylamine hydrochloride. Hydroxylamine sulfate and hydroxylamine hydrochloride are particularly preferable from the viewpoint of stability and price. The hydroxylamine or salts may be in the form of an aqueous solution or an organic solution such as in methanol.

Examples of dihydrocarbyl carbonate of formula (I) are dialkyl carbonates such as dimethyl carbonate, diethyl carbonate, n-propyl carbonate or diisopropyl carbonate; dicycloalkyl carbonates such as dicyclopentyl carbonate, dicyclohexyl carbonate or dicycloheptyl carbonate ; diaryl carbonates such as diphenyl carbonate, ditolyl carbonate or dixylyl carbonate ; and diaralkyl carbonates such as dibenzyl carbonate or diphenetyl carbonate. In case of, for example, preparing methyl N,O-dimethylhydroxycarbamate, dimethyl carbonate is used.

Examples of a base are hydroxides of alkali metals or alkaline earth metals such as sodium hydroxide, potassium hydroxide, lithium hydroxide, cesium hydroxide and barium hydroxide; hydrogencarbonates of alkali metals such as sodium hydrogencarbonate and potassium hydrogen carbonate; carbonates of alkali metals or alkaline earth metals such as sodium carbonate, potassium carbonate and barium carbonate; and aluminate compounds such as sodium aluminate and potassium aluminate. These may be used independently or in combination. Among these basic compounds, sodium hydroxide is particularly preferable from the viewpoint of price. It is preferred that these basic compounds are used in the form of an aqueous solution. However, they may be used as in the solid state or may be dissolved in an organic solvent such as an alcohol.

Examples of the alkylating agent are dialkylsulfuric acid esters such as dimethyl sulfate and diethyl sulfate; alkyl chlorides such as methyl chloride, ethyl chloride, propyl chloride and butyl chloride; alkyl bromides such as methyl bromide, ethyl bromide, propyl bromide and butyl bromide; and alkyl iodidessuch as methyl iodide, ethyl iodide, propyl iodide and butyl iodide. Dialkylsulfuric acid ester is preferable from the viewpoint of price. In case, for example, of preparing methyl N,O-dimethylhydroxycarbamate, it is preferred to use dimethyl sulfate.

As a reaction solvent , it is preferred to use water. However, inert organic solvents, for example, ethers such as diethyl ether and tetrahydrofuran; and aromatic compounds such as benzene and toluene may be used independently, in combination or as a two-layer system.

The molar ratio of the hydroxylamine or its salt to the dihydrocarbyl carbonate of formula (I) is generally 1:0.5 - 1:2, preferably 1:1 - 1:1.5 more preferably 1:1 - 1:1.3.

The molar ratio of the hydroxylamine or its salt to the alkylating agent is generally 1:2 or more. In case of using dimethyl sulfate as the alkylating agent, the molar ratio is preferably 1:2 - 1:4, more preferably 1:2 - 1:2.5.

There is no particular restriction on the quantity of the base to be used. However, it is preferably used in such a quantity as to control the pH of the reaction solution to within the range given later.

Although there is no particular restriction on the method for adding the starting materials, a method comprising firstly loading the hydroxylamine or its salt and a reaction solvent into a reactor and subsequently supplying the dihydrocarbyl carbonate of formula (I) and a basic compound simultaneously is preferable because the reaction temperature and the pH of the reaction solution can thus be controlled to be constant. In case of using the sulfate or hydrochloride of hydroxylamine as a starting material, it is preferred to neutralize the salt by adding the base before supplying the dihydrocarbyl carbonate of formula (I).

In the dialkylation it is preferred to adopt a method for simultaneously supplying the alkylating agent and the base in order to control the reaction temperature and the pH of the reaction solution to be constant.

The hydroxycarbamic acid ester of formula (II) may be is lated as an intermediate on the way, or the two reactions, carbamoylation and dialkylation, may be continuously carried out using the same reactor without isolating the intermediate. In consideration of the reaction time and cost of extraction solvent, it is preferred that the two reactions are carried out using the same reactor without performing the isolation on the way.

The reaction temperature range at the time of carbamoylation or dialkylation is generally from -20 to 80°C, preferably from -10 to 40°C, more preferably from 0 to 25°C.

The pH range of the reaction solution at this time is generally 7 - 14, preferably 11 - 14, more preferably 12 - 14.

A combination of a reaction temperature of 5 ± 5°C with a reaction solution pH of 12 - 13 is optimum in case of industrially preparing the desired product not only because the yield is high but also because the reaction temperature and the reaction solution pH can be easily controlled.

The reaction pressure is generally atmospheric pressure or higher. In case that an alkylating agent vaporizes under the reaction conditions, it is required to carry out the reaction under pressure.

Examples of the N,O-dialkylhydroxycarbamic acid esters of formula (III) obtained are N,O-dimethylhydroxycarbamic acid esters such as methyl N,O-dimethylhydroxycarbamate, ethyl N,O-dimethylhydroxycarbamate, propyl N,O-dimethylhydroxycarbamate or butyl N,O-dimethylhydroxycarbamate; N,O-diethyl hydroxycarbamic acid esterssuch as methyl N,O-diethylhydroxycarbamate, ethyl N,O-diethylhydroxycarbamate, propyl N,O-diethylhydroxycarbamate or butyl N,O-diethylhydroxycarbamate ; N,O-dipropylhydroxycarbamic acid esters such as methyl N,O-dipropylhydroxycarbamate, ethyl N,O-dipropylhydroxycarbamate, propyl N,O-dipropylhydroxycarbamate or butyl N,O-dipropylhydroxycarbamate; and N,O-dibutylhydroxycarbamic acid esters such as methyl N,O-dibutylhydroxycarbamate, ethyl N,O-dibutylhydroxycarbamate, propyl N,O-dibutylhydroxycarbamate or butyl N,O-dibutylhydroxycarbamate. Methyl N,O-dimethylhydroxycarbamate is particularly desired.

According to the present process for preparing N,O-dialkylhydroxycarbamic acid esters, the dihydrocarbyl carbonate of formula (I) is not only difficult to hydrolyze but is also unreactive with the hydroxy group of hydroxycarbamic acid esters formed as intermediates. Therefore, the desired N,O-dialkylhydroxycarbamic acid ester can be obtained in high yields.

Although it is possible to carry out the recovery of the N,O-dialkylhydroxycarbamic acid ester of formula (III) by extraction with an organic solvent such as chloroform, methylene chloride or ethylene dichloride with subsequent fractional distillation, the present inventors have found that the ester has an azeotropic found point with the water existing in the reaction system and that unreacted starting materials or O-alkylhydroxycarbamic acid esters of formula (IV): R²ONHCOOR¹, wherein R¹ and R² are as defined as above, existing as impurities did not become contained in the azeotropic composition when the desired ester of formula (III) was directly distilled.

Thus the N,O-dialkylhydroxycarbamic acid ester can be recovered by azeotropically distilling the N,O-dialkylhydroxycarbamic acid ester with water, without extracting the ester from the reaction mixture using an organic solvent as described above.

The N,O-dialkylhydroxycarbamic acid ester is azeotropically distilled with water from a solution containing inorganic salts such as sodium sulfate and sodium chloride, by-products formed in the preparation of the N,O-dialkylhydroxycarbamic acid ester, unreacted reagents and O-alkylhydroxycarbamic acid ester formed as impurities. This process selectively and easily purifys and distills said ester.

The quantity of water necessary to form an azeotrope is generally the compositional quantity of an azeotrope of water and N,O-dialkylhydroxycarbamic acid ester. That is, under atmospheric or reduced pressure distillation conditions, the quantity of water to ester by weight ratio is generally 1.0 :1 or more, preferably 1.0 - 1.1:1.

The supply of water to be used for forming the above azeotrope is generally carried out by adding the quantity necessary for azeotropic composition at the time of distillation. However, water which already exists in the mixture may be used. The mixture generally is a homogeneous system containing organic compounds such as an alcohol and inorganic compounds. However, it does not matter if the mixture is a heterogenous system containing other organic and inorganic compounds.

As a distillation apparatus for the above azeotrope composition, it is generally desirable to use an apparatus having a distillation column and having a fractionally distilling function such as an Oldershow distillation column, that is an apparatus which enables selective recovery of azeotropic compositions alone. However, it is possible to use a batch system apparatus and a continuous system apparatus, each having a function of simultaneously recovering other compounds and azeotropic compositions.

Pressure conditions in the above distillation may be any of atmospheric pressure, pressurization or reduced pressure. Generally, atmospheric pressure or reduced pressure is adopted. The pressure range is preferably 1 - 760 mmHg, more preferably 10 - 250 mmHg. The distillation is generally carried out at a boiling point temperature where an azeotrope is formed at a set pressure. The distillation temperature, though it ranges preferably from 20 to 160°C, more preferably from 30 to 80°C, may exceed the boiling point temperature where an azeotrope is formed at a set pressure.

An N,O-dialkylhydroxylamine of formula (V): R²ONHR² wherein R² is as defined above can be prepared by hydrolyzing the N, O-dialkylhydroxyearbamic acid ester of formula (III) with an acid such as hydrochloric acid or sulfuric acid; Free anhydrous N,O-dialkylhydroxylamine can also be obtained without forming the N,O-dialkylhydroxylamine salt by carrying out hydrolysis using a catalystic quantity of an alkali instead of acids according to an industrial method.

The present invention will now be further described in the following Examples and Comparative Examples.

### Example 1

A stirrer was placed in a 200-ml four-neck flask equipped with a pH meter electrode, a thermometer, an injector and a feed pump supply pipe. Then, 8.2g (95 mmol) of 95% hydroxylamine sulfate (produced by Wako Junyaku Co., Ltd.) and 25ml of water were added to the flask, and the flask was cooled to an internal temperature of 5°C. In an atmosphere of nitrogen, a 50% sodium hydroxide solution was added dropwise to the above solution by the injector that the pH of the reaction solution reached 13. Maintaining the internal temperature at 5°C and the pH of the reaction solution at 13, 10g (108 mmol) of dimethyl carbonate (produced by Wako Junyaku Co., Ltd.) and a 50% sodium hydroxide solution were simultaneously supplied into the flask with the injector and the feed pump respectively over a period of 40 minutes. After the completion of supply, the mixture was stirred for 2 hours under the above conditions. Thereafter, maintaining the reaction temperature at 5°C and the pH of the reaction solution at 13, 27.8g (209 mmol) of 95% dimethyl sulfate (produced by Wako Junyaku Co., Ltd.) and a 50% sodium hydroxide solution were simultaneously supplied into the flask with the feed pipe and the injector respectively over a period of 1 hour. After the completion of supply, the mixture was stirred for 3 hours under the above conditions. Then, chloroform was added to the reaction solution to extract methyl N,O-dimethylhydroxycarbamate. By quantitative analysis of the extract by gas chromatography, it was found that 11.0g (yield, 97%) of methyl N,O-dimethylhydroxycarbamate was formed.

### Examples 2 - 8

Reactions were carried out under the same conditions as in Example 1, except that only the pH of the reaction solution was changed. Results of the experiments are given in Table 1.

**Table 1**

| No. of Example | pH in Carbamoylation | pH in Dimethylation | Yield (%) |
|---|---|---|---|
| 2 | 12.5 | 12.5 | 98 |
| 3 | 12 | 12 | 90 |
| 4 | 12 | 13 | 94 |
| 5 | 13 | 12 | 94 |
| 6 | 13.5 | 13.5 | 92 |
| 7 | 14 | 13 | 91 |
| 8 | 14 | 14 | 84 |

### Example 9

4.55g (50 mmol) of methyl hydroxycarbamate, which had been isolated by adjusting the reaction solution after the carbamoylation in Example 1 to pH 7, evaporating the reaction solution to dryness, extracting the dried product with ethyl acetate and then subjecting the product to column chromatography, and 15ml of water were loaded into the same reaction device as in Example 1. The internal temperature of the device was then cooled to 5°C. In an atmosphere of nitrogen, a 50% sodium hydroxide solution was added dropwise to the mixture from an injector so as to adjust the the reaction solution to pH 13. Maintaining the internal temperature at 5°C and the pH of the reaction solution at 13, 13.9g (105mmol) of 95% dimethyl sulfate (produced by Wako Junyaku Co., Ltd.) and a 50% sodium hydroxide solution were simultaneously supplied to the device with the feed pump and the injector respectively over a period of 1 hour. After the completion of supply, the mixture was stirred for 5 more hours under the above conditions. Then, chloroform was added to the reaction solution to extract methyl N,O-dimethylhydroxycarbamate. By quantitative analysis of the extract by gas chromatography, it was found that 5.2g (yield, 87%) of methyl N,O-dimethylhydroxycarbamate was formed.

### Comparative Example 1

A stirrer was placed in a 50-ml four-neck flask equipped with a thermometer and an injector. Then, 1.67g (20 mmol) of 95% hydroxylamine sulfate (produced by Wako Junyaku Co., Ltd. ) and 5ml of water were added to the flask. The flask was then cooled to an internal temperature of 5°C. In an atmosphere of nitrogen, 2.12g (22 mmol) of 98% methyl chloroformate was added to the above solution, followed by dropping 6.2ml of a 10N sodium hydroxide solution from the injector over a period of 1 hour while maintaining the reaction temperature at 8°C or less. Maintaining the internal temperature within the range of 2 - 5°C, the mixture was stirred for 3 more hours. Thereafter, 5.84g (44 mmol) of 95% dimethyl sulfate (produced by Wako Junyaku Co., Ltd. ) was added to the reaction solution, followed by dropping 2.4ml of 10N sodium hydroxide solution using the injector over a period of 30 minutes. After the completion of supply, the reaction temperature was raised to 15 - 20°C, at which the mixture was stirred for 3 hours. Thereafter, the reaction solution was subjected to quantitative analysis by liquid chromatography. After repeating the same reaction as above twice, 0.87g (yield, 37%) and 1.15g (yield, 47%) of methyl N,O-dimethylhydroxycarbamate were formed respectively.

### Example 10

The reaction was carried out under the same conditions as in Comparative Example 1, except that dimethyl carbonate was used instead of methyl chloroformate and the quantity of a 10N sodium hydroxide solution to be dropped after the addition of dimethyl carbonate was changed to 4ml. As a result, the yield of methyl N,O-dimethylhydroxycarbamate was 72%.

### Comparative Example 2

A 50-ml four-neck flask equipped with a thermometer and two injectors was loaded with 1.82g (20 mmol) of methyl hydroxycarbamate and 6m of water. Then, 0.77ml (10 mmol) of 98% methyl chloroformate and lm (10 mmol) of a 10N sodium hydroxide solution were simultaneously dropped into the flask over a period of 20 minutes from the injectors while maintaining the reaction temperature at 5°C. Maintaining the internal temperature at 5°C, the mixture was stirred for 1 more hour. As a result of qualitatively analyzing the reaction mixture by liquid chromatography, it was found that methyl O-methoxycarbonylhydroxycarbamate was formed. The peak area ratio of methyl hydroxycarbamate as the raw material to methyl O-methoxycarbonylhydroxycarbamate in liquid chromatography was 62:35.

### Comparative Example 3

The reaction was carried out under the same condition as in Comparative Example 2, except that dimethyl carbonate was used instead of methyl chloroformate. Methyl O-methoxycarbonylhydroxycarbamate was not formed at all.

### Example 11

41.03g (0.485 mol) of 95% hydroxylamine sulfate and 125m of water were added to a flask. The internal temperature of the flask was then cooled to 5°C. In an atmosphere of nitrogen, a 50% sodium hydroxide solution was dropped into the above solution from a dropping funnel such that the pH of the reaction solution reached 12. Then, maintaining the internal temperature at 5°C and the pH of the reaction solution within the range of 12 - 13, 49.5g (0.52 mol) of 98% dimethyl carbonate and a 50% sodium hydroxide solution were simultaneously supplied into the flask from the dropping funnels over a period of 40 minutes. After the completion of supply, the mixture was stirred for 30 minutes under the same conditions. Thereafter, maintaining the reaction temperature within the range of 5 - 8°C and the pH of the reaction solution within the range of 12 - 13, 139.0g (1.05 mol) of 95% dimethyl sulfate and a 50% sodium hydroxide solution were simultaneously supplied into the flask from dropping funnels over a period of 1.5 hours. After the completion of supply, the mixture was stirred for 3 hours under the same conditions. Then, the reaction temperature was raised to 50°C, and the reaction solution was stirred for 30 minutes while maintaining the pH thereof at 7.3 by dropping a saturated sodium hydrogencarbonate solution to decompose excessive dimethyl sulfate. After cooling to room temperature, the reaction solution was quantitatively analyzed by gas chromatography. It was found that 54.9g (yield, 95%) of methyl N,O-dimethylhydroxycarbamate was formed. Then, a five-stage Oldershow distillation column was attached to the flask to distill the reaction solution under a reduced pressure of 50 - 80 mmHg and at a boiling point of 40 - 50°C. 181.3g (purity, 29.0%; isolation yield, 92.5%) of an azeotropic distillate with water containing methanol and methyl N,O-dimethylhydroxycarbamate was obtained. N,O-dimethylhydroxylamine hydrochloride as the final product can be obtained by adding hydrochloric acid to this azeotropic distillate to hydrolyze the same and then evaporating the hydrolysate to dryness.

### Example 12

The reaction was carried out in the same manner as in Example 11, except that the distillation was carried out at atmospheric pressure and at a boiling point of 90 - 105°C instead of a reduced pressure of 50 - 80 mmHg and a boiling point of 40 - 50°C. 183.1g (purity, 24%; isolation yield, 77%) of an azeotropic distillate with water containing methanol and methyl N,O-dimethylhydroxycarbamate was obtained.

## Claims

1. A process for preparing an N,O-dialkylhydroxycarbamic acid ester of formula (III) 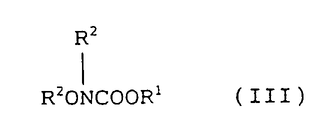 wherein R¹ is a hydrocarbon group, and R² is lower alkyl, said process comprising reacting hydroxylamine of formula NH₂OH or a salt thereof with a dihydrocarbyl carbonate of formula (I)
R¹OCOOR¹ (I)
wherein R¹ is as defined above,
in the presence of a base, and alkylating the resulting hydroxycarbamic acid ester of formula (II)
HONHCOOR¹ (II)
wherein R¹ is as defined above,
with an alkylating agent in the presence of a base.

2. A process according to claim 1, wherein the dihydrocarbyl carbonate of formula (I) is dimethyl carbonate, the hydroxycarbamic acid ester of formula (II) is methyl hydroxycarbamate and the N,O-dialkylhydroxycarbamic acid ester of formula (III) is methyl N,O-dimethylhydroxycarbamate.

3. A process according to claim 1 or 2, wherein the reaction temperatures in carbamoylation and alkylation are 5 ± 5°C and the pHs of the reaction solutions are from 12 to 13.

4. A process according to claim 1, 2 or 3, wherein the N,O-dialkylhydroxycarbamic acid ester of formula (III) is recovered by azeotropic distillation with water.

## Patentansprüche

1. Verfahren zur Herstellung eines N,O-Dialkylhydroxycarbarninsäureesters der Formel (III) 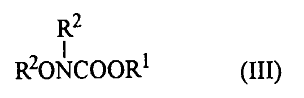 in der R¹ ein Kohlenwasserstoffrest ist, und R² ein Niederalkylrest ist, wobei das Verfahren die Umsetzung eines Hydroxylamins der Formel NH₂OH oder dessen Salzes mit einem Kohlensäuredikohlenwasserstoffester der Formel (I)
R¹OCOOR¹ (I)
in der R¹ wie vorstehend definiert ist, in Gegenwart einer Base und das Alkylieren des entstandenen Hydroxycarbaminsäureesters der Formel (II)
HONHCOOR¹ (II)
in der R¹ wie vorstehend definiert ist, mit einem Alkylierungsmittel in Gegenwart einer Base umfaßt.

2. Verfahren gemäß Anspruch 1, wobei der Kohlensäuredikohlenwasserstoffester der Formel (I) Kohlensäuredimethylester ist, der Hydroxycarbaminsäureester der Formel (II) Hydroxycarbaminsäuremethylester ist, und der N,O-Dialkylhydroxycarbarninsäureester der Formel (III) N,O-Dimethylhydroxycarbaminsäuremethylester ist.

3. Verfahren gemäß Anspruch 1 oder 2, wobei die Reaktionstemperaturen bei der Carbamoylierung und Alkylierung 5 ± 5°C betragen, und die pH-Werte der Reaktionslösungen 12 bis 13 sind.

4. Verfahren gemäß Anspruch 1, 2 oder 3, wobei der N,O-Dialkylhydroxycarbaminsäureester der Formel (III) durch azeotrope Destillation mit Wasser gewonnen wird.

## Revendications

1. Procédé qui consiste à préparer un ester d'acide N,O-dialkylhydroxycarbarmique de formule (III) : 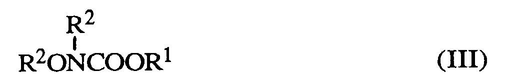 dans laquelle R¹ représente un groupe hydrocarboné, et R² représente un groupe alkyle inférieur, ledit procédé comprenant le fait de faire réagir une hydroxylamine de formule NH₂OH ou un sel de cette dernière avec un carbonate de dihydrocarbyle de formule (I) :
R¹OCOOR¹ (I)
dans laquelle R¹ a la même définition que précédemment,
en présence d'une base, et le fait d'alkyler l'ester d'acide hydroxycarbamique résultant de formule (II) :
HONHCOOR¹ (II)
dans laquelle R¹ a la même définition que précédemment, avec un agent d'alkylation en présence d'une base.

2. Procédé conforme à la revendication 1, dans lequel le carbonate de dihydrocarbyle de formule (I) est le carbonate de diméthyle, l'ester d'acide hydroxycarbamique de formule (II) est l'hydroxycarbamate de méthyle et l'ester d'acide N,O-dialkylhydroxycarbamique de formule (III) est le N,O-dialkylhydroxycarbamate de méthyle.

3. Procédé conforme à la revendication 1 ou 2, dans lequel les températures de réaction dans la carbamylation et l'alkylation valent 5 ± 5° C et les pH des solutions de réaction sont compris entre 12 et 13.

4. Procédé conforme à la revendication 1, 2 ou 3, dans lequel on récupère l'ester d'acide N,O-dialkylhydroxycarbamique de formule (III) par distillation azéotropique avec de l'eau.
